# EUROPEAN PATENT SPECIFICATION

(11) **EP 2 092 919 B1**
(45) Date of publication and mention of the grant of the patent: **01.02.2012**
(21) Application number: 08151819.3
(22) Date of filing: 22.02.2008
(51) Int. Cl.: A61F 13/15, A61F 13/56

(54) **Absorbent article**
Saugfähiger Artikel
Article absorbant

(43) Date of publication of application: 26.08.2009
(73) Proprietor: Attends Healthcare AB, 578 24 Aneby (SE)
(72) Inventor: Gerbino, Jenny, 330 12, Forsheda (SE); Persson, Christer, 578 31, Aneby (SE); Svensson, Ida, 554 50, Jönköping (SE); Håkansson, Mikael, 573 37, Tranås (SE); Lindqvist, Arne, 152 57, Södertälje (SE)
(74) Representative: Fritsche, Daniel

(56) References cited:
- EP-A- 0 471 385
- EP-A- 0 931 530
- GB-A- 2 273 279
- US-A- 4 690 680
- US-A- 5 484 636
- US-A- 5 670 004

## Description

### Technical Field

The present disclosure relates to an absorbent article such as a sanitary napkin or an incontinence guard, and especially to an absorbent article for attaching to and wearing in an undergarment of a user.

### Background

A wide variety of absorbent articles for collecting body fluids are known in the art. Generally, such absorbent articles comprise a central absorbent core disposed between a topsheet, defining a surface for facing the body of a user, and a backsheet, defining a surface for facing away from the user's body.

Moreover, absorbent articles for wearing in the undergarment of a user generally comprise some kind of fastening means, intended to secure the absorbent article to a body-facing surface of e.g. a crotch portion of the undergarment. The absorbent article is thereby retained in place against the perineal region of the user, as long as he or she wears the undergarment to which the absorbent article is secured.

The fastening means may e.g. be a layer of adhesive material provided on the surface of the absorbent article that faces away from the body of the user, i.e. the undergarment-facing surface.

Prior to use of the absorbent article, the fastening means is preferably covered by a peelable protective layer that prevents soiling of the fastening means and entanglement of the absorbent article when it is wrapped in a package.

The user may experience difficulties in removing the peelable protective layer from the fastening means before attaching the absorbent article to his or her undergarment. US 2007/0197991 discloses an absorbent article that is intended to overcome this drawback. The absorbent article has a peelable protective layer including a pull tab. The pull tab contacts the peelable protective layer to promote separation of the peelable protective layer from the fastening means when the pull tab is being pulled.

US 2007/0197991 discloses an absorbent article having a fastening means that forms one single adhesive zone, covering substantially all of the undergarment-facing surface of the backsheet.

Absorbent articles where the adhesive material covers a major part of the undergarment-facing surface of the backsheet, such as described in US 2007/0197991, have two essential drawbacks.

Firstly, such a large area of the adhesive material may result in an absorbent article that is tricky to attach to the undergarment since the absorbent article will easily entangle while attaching it to the undergarment, particularly if the absorbent article is a large article, e.g. considerably longer than a panty liner. This is also true if there are several adhesive zones spread all over the undergarment-facing surface of the backsheet.

Furthermore, when the absorbent article is attached to the user's undergarments such that substantially all of the undergarment-facing surface of the backsheet is in contact with the undergarment, the fitting properties of the absorbent article may be placed in disadvantage, especially if the undergarment does not have a very good fit. If e.g. there is a distance between the body of the user and the crotch region of the undergarment, there will also be a distance between the body of the user and the portion of the absorbent article that is attached to the crotch region of the undergarment, since the absorbent article is so closely connected to the undergarment. Impaired fitting properties result in an increased risk for leakage, and the user will probably regard the absorbent article as uncomfortable.

EP 0 550 736 discloses an absorbent article having a fastening means that consists of several adhesive zones, which zones are located only in the end portions of the absorbent article. This solution provides an absorbent article with a central portion that is capable of decoupling from the users undergarment for improved body contact and better fit. One single protective layer covers all the adhesive zones, and consequently also a major part of the undergarment-facing surface of the backsheet of the absorbent article.

A drawback with an absorbent article according to EP 0 550 736 becomes obvious prior to use of the absorbent article. Since the middle portion of the protective layer is not attached to any adhesive material, this portion will not abut on the undergarment-facing surface of the absorbent article, but will slack such that that a space appears between the undergarment-facing surface of the absorbent article and the protective layer. Extraneous objects will easily wedge into this space, causing the protective layer to come off unintentionally. As a result, the fastening means will be left unprotected and can stick to any adjacent surface, and the absorbent article might entangle or get stuck. The fastening means can also be soiled, resulting in impaired adhesive properties. This problem will especially come up for absorbent articles that are not folded prior to use or absorbent articles that prior to use are unfolded from a previously folded position.

The space that appears between the undergarment-facing surface of the absorbent article and the protective layer may also give the absorbent article an unattractive appearance.

EP 931530 describes an incontinence pad having a part of spaced a part securement patches positioned adjacent each end edge, and a unitary release strip bridging the securement patches.

Users may wish to carry extra absorbent articles with them in e.g. a purse or a pocket. Since an individual absorbent article may become soiled while being transported from place to place, many absorbent articles are folded prior to being packaged. The absorbent article is preferably folded in such a way that the topsheet, which is intended to face the body, is directed inwardly, and thus protected from soiling. Several folded absorbent articles may then be packaged together in one common package. Absorbent articles may also be folded and then individually packaged. Before using a folded absorbent article, the user will have to unfold it, revealing the drawbacks described above.

US5484636 and GB2273279 both describe an absorbent article that is in a folded condition before it is used. US5484636 has adhesive patches arranged on the backsheet and a wrapper wrapped around the article functioning as a protective layer. GB2273279 has adhesive areas arranged on the backsheet and a wrapper having a peelable layer at a position that is opposed to each adhesive area.

An absorbent article according to EP 0 550 736 has one more drawback. Unnecessarily large amounts of protective layer paper are used during manufacture of the absorbent article, and the production cost becomes greater than what is necessary.

### Summary

It is an object of the present invention to provide an improved solution that alleviates at least some of the mentioned drawbacks with present absorbent articles.

The invention is based on the discovery that if a protection means extends beyond its corresponding fastening means proximal to a folding, there is an increased risk that the protection means will come loose unintentionally.

According to the invention, an absorbent article according to the preamble of claim 1, is arranged such that each fastening means has a separate protection means for covering the fastening means, each protection means being arranged with a projecting portion for facilitating removal of the protection means from the fastening means, and that a portion of each protection means, proximal to the folding line is coterminous with a corresponding portion of the corresponding fastening means in a direction towards the folding line. Thereby, a product is achieved, for which a low amount of protection means material is needed, and for which there is a reduced risk of unintentional removal of the protection means, which absorbent article is easy to attach to the undergarment.

The projecting portion is a portion of the protection means which projects beyond an edge of the corresponding fastening means when the protection means and the fastening means are stuck together prior to use of the absorbent article. This projecting portion is suitable for using as a pull-tab which the user can grab and pull for removal of the protection means from the fastening means.

The absorbent article according to the present invention is arranged to be kept in a folded configuration prior to use. The positions or the areas of the absorbent article where the absorbent article is intended to be folded are called folding lines. Keeping the absorbent article in a folded configuration prior to use facilitates packaging and transportation since the bundle that the folded absorbent article defines is easier to package or carry about in e.g. a pocket than an unfolded absorbent article.

When the absorbent article is folded such that the body-facing surface of the main body portion is directed inwardly, this surface is also protected from soiling prior to use of the absorbent article.

If a protection means has a projecting portion, the curvature of this projecting portion will easily deviate from the curvature of the main body portion of the absorbent article, if the projecting portion is located close to a folding. Consequently the projecting portion of the protection means will protrude outside the edges of a package defined by the folded main body portion. Such protruding portion may touch any surrounding surface or object, causing an unintentional partial removal of the portion of the protection means that covers the fastening means. As a result, the fastening means will be left partly unprotected and the absorbent article might entangle or get stuck to any surrounding surface. The fastening means can also get soiled and loose its adhesive properties.

By providing an absorbent article such that a portion of each protection means, proximal to the folding line, is coterminous with a corresponding portion of the corresponding fastening means in a direction towards the folding line, as mentioned in the characterizing portion of claim 1, such unintentional removal of the protection means is prevented, since there is no projecting portion of the protection means on a side of the fastening means that is adjacent to a folding line.

By placing the fastening means and the protection means only in the end regions of the absorbent article, as mentioned in claim 3, a better fit of the absorbent article to a user may be achieved and consequently there is a reduced risk for leakage. Since a central portion of the absorbent article is not attached to the body-facing surface of the undergarment, this central portion is capable of separating from the undergarment, for improved body contact. This is useful especially if the undergarment to which the absorbent article is secured does not have a very good fit.

Furthermore, a relatively large absorbent article having a fastening means that covers substantially all of the undergarment-facing surface of the main body portion, or having several fastening means spread all over the same surface, may be tricky to attach to the undergarment since there is so much adhesive material that the absorbent article might entangle while attaching it to the undergarment. This problem is also solved by an absorbent article according to claim 3, since the fastening means are only located in the end regions of the absorbent article.

Furthermore, if the absorbent article is relatively large, the manufacturing cost is reduced since less material for the fastening means and the protection means is used than if substantially all of the body-facing surface of the main body portion is covered by an fastening means and a protection means.

Also, since the protection means and the fastening means are only arranged in the end regions, the risk of extraneous objects wedging in between the protection means and the fastening means, causing the protective layer to come off unintentionally, is low.

By designing the protection means such that the projecting portion is placed in an end of the protection means that is distal to the folding lines of the absorbent article, according to claim 4, the procedure of attaching the absorbent article to the undergarment is facilitated. The user can release one of the protection means, place the absorbent article in a wanted position in the crotch region of the undergarment, and attach the unprotected fastening means to the undergarment. When the absorbent article is in the desired position, the projecting portion of the remaining protection means is easily accessible, since it is located distal to the folding lines and consequently proximal to an end of the absorbent article that is located proximal to the waist portion of the undergarment to which the absorbent article is attached. The projecting portion is also directed towards the waist portion of the undergarment. The position and direction of the projecting portion ensures that the user can effortlessly grab the projecting portion and remove the protection means.

According to the embodiment described in claim 6, the portion of each protection means proximal to the folding line, and the corresponding portion of the corresponding fastening means each has a rounded shape. Thereby, corners are avoided close to the folding lines, whereby the risk of unintentionally peeling off the protection means is low.

According to the embodiment of claim 7, the portion of each protection means, proximal to the folding line, and the corresponding portion of the corresponding fastening means each has at least one edge that is substantially parallel to its most adjacent folding line. Thereby, a potential stress in a direction of separating the protection means from the fastening means due to the folding of the product, which stress would be directed to the end of the protection means arranged closest to the folding line is spread out over a large part of the protection means, whereby the risk of unintentionally peeling off the protection means is low.

### Brief Description of the Drawings

The invention will in the following be described in more detail with reference to the enclosed drawings, wherein:
Fig. 1 is a planar view of the undergarment-facing side of an absorbent article according to the invention, illustrating the fastening means and the protection means.
Fig. 2 is a side view of an absorbent article according to the present invention, in a folded condition.
Fig. 3 is a cross-sectional view of the absorbent article in fig. 1, taken along line III-III in fig.1.

### Description of Embodiments

The present invention will be described more fully hereinafter with reference to the accompanying drawings, in which preferred embodiments of the invention is shown. This invention may, however, be embodied in many different forms and should not be construed as limited to the embodiments set forth herein; rather, these embodiment are provided so that this disclosure will be thorough and complete, and will fully convey the scope of the invention to those skilled in the art. In the drawings, like numbers refer to like elements.

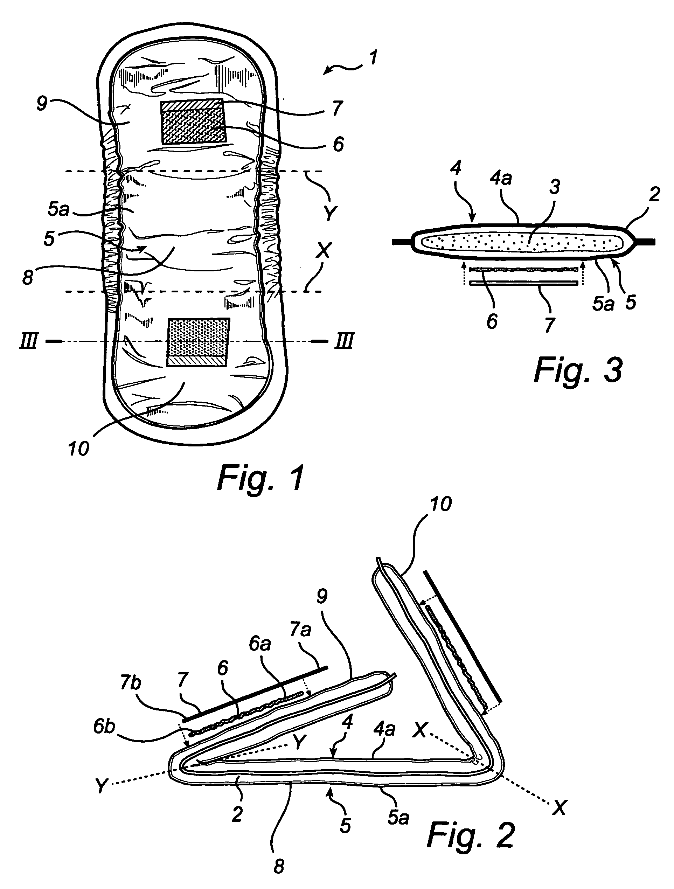

Shown in figures 1-3 is an absorbent article 1, in accordance with a specific non-limiting example of presentation of the present invention.

The absorbent article 1 comprises a main body portion 2, which has a central absorbent core 3, positioned between a topsheet 4 and a backsheet 5.

The topsheet 4 is preferably fluid-permeable and has a surface 4a that is intended to face, and abut on, the body of a user during use of the absorbent article. This surface 4a is hereafter called the body-facing surface 4a of the main body portion 2. The backsheet 5 is suitably fluid-impermeable and has a surface 5a that is intended to face away from the body of the user during use of the absorbent article. This surface 5a will face the undergarment to which the absorbent article is secured, and will hereafter be called the undergarment-facing surface 5a of the main body portion 2.

Furthermore, an absorbent article 1 according to the present invention comprises a plurality of fastening means 6 for securing the absorbent article 1 to a body-facing surface of the user's undergarment. The absorbent article 1 is thereby retained in place against the perineal region of the user, as long as he or she wears the undergarment to which the absorbent article is secured.

A fastening means 6 may e.g. be a layer of adhesive material, i.e. an adhesive layer, provided on the undergarment-facing surface 5a of the backsheet 5. In the embodiment shown in the figures, the adhesive layers 6 are two rectangular adhesive zones, located in a first end region 9 and a second end region 10 of the absorbent article. The article also has a central region 8 arranged between the first and the second end region. There may also be several adhesive zones of various shapes.

Each adhesive layer 6 has a corresponding protection means 7 arranged to cover the adhesive layer prior to use of the absorbent article 1. Each adhesive layer has its own protection means. I.e. each adhesive layer has a separate protection means, separate from a protection means of another adhesive layer. The protection means 7 is e.g. a peelable protective layer 7 fabricated by e.g. silicone coated paper or plastic. The protective layer 7 prevents soiling of the adhesive layer and entanglement of the absorbent article when it is wrapped in a package.

Users may wish to carry extra absorbent articles with them in e.g. a purse or a pocket. In order to facilitate packaging and transportation of absorbent articles, and for protecting the body-facing surface 4a of the articles, the absorbent article according to the present invention is intended to be stored in a folded configuration prior to use. The absorbent article according to the embodiment shown in figures 1-3 is folded twice, forming a tri-folded configuration, as shown in figure 2. In figure 1, folding lines X-X, Y-Y along which the absorbent article is folded, are shown. Other folding configurations are also possible. The absorbent article is preferably folded in such a way that the body-facing surface 4a of the main body portion 2, is directed inwardly, and thus protected from soiling. Several folded absorbent articles may then be packaged together in one common package. Absorbent articles may also be folded and then individually packaged in separate wrappings before packaging them together in a common package. When folded according to the embodiment shown in the figures, the folding lines X-X, Y-Y will define the extension of the central region 8 and the end regions 9, 10.

According to the present invention, the surface area of the protective layer 7 is larger than the surface area of the adhesive layer 6 that it protects. Consequently, the protective layer covers the adhesive layer completely and there is also a portion 7a of the protective layer that projects beyond the borders of the adhesive layer, in a direction away from the folding lines. This projecting portion 7a is arranged for facilitating removal of the protective layer from its corresponding adhesive layer, i.e. it can be designed to function as a pull tab, for facilitating removal of the protective layer from the adhesive layer. The projecting portion shown in the embodiment of the figures has a rectangular shape which is an extension of a rectangular shape of a main portion of the protective layer, i.e. the projecting portion has a similar extension in a direction parallel to a folding line of the article than the main portion of the protective layer. Although, the projecting portion can have any shape suitable for functioning as a pull-tab, such as a more rounded shape. The projecting portion can also have an extension in a direction parallel to a folding line that is considerably smaller than the extension direction parallel to a folding line of the protective layer. The projecting portion 7a may also be arranged such that it has at least partly an extension perpendicular to the undergarment-facing surface of the article.

When the absorbent article is folded, as shown in figure 3, a first portion 6a of the adhesive layer and the projecting portion 7a of the protective layer will be distal to the folding line, and a second portion 6b, 7b of the adhesive layer 6 and protective layer 7 will be proximal to the folding line X-X, Y-Y.

According to the present invention, the edge of the adhesive layer 6 that is proximal to the closest folding line coincides with the edge of the protective layer 7 that is proximal to the closest folding line, such that there is no portion of the protective layer that projects beyond the adhesive layer in a direction towards the closest folding line X-X; Y-Y. Thereby, the second portion 6b of the adhesive layer 6 and the second portion 7b of the protective layer 7 are coterminous at least in the direction towards the closest folding line X-X, Y-Y.

According to the embodiment shown in the figures, each adhesive layer 6 and its corresponding protective layer 7 has a rectangular shape where the edge proximal to the folding line is substantially parallel to the folding line. Any other shape of protective layer and adhesive layer can be used, but by making the coinciding edges of the adhesive layer and its corresponding protective layer substantially parallel to the folding line results in a low risk of an unintentional peeling off of the protective layer close to the folding line.

In an alternative embodiment of the invention, each adhesive layer and its corresponding protective layer has a rounded shape close to the folding line. Thereby, corners are avoided close to the folding line, whereby the risk of an unintentional peeling off of the protective layer close to a folding line is low.

According to an embodiment of the present invention, the adhesive layer 6 is arranged into a striped pattern.

In the embodiment shown in the figures, the projecting portion 7a of the protective layer 7 is only located at one side of the adhesive layer 6, i.e. in the portion distal to the closest folding line. However, there may be projecting portions in other directions as well. What is important is that no portion of the protective layer projects beyond the side of an adhesive layer that is closest to a folding line when the absorbent article is in a folded condition.

In the drawings and specification, there have been disclosed preferred embodiments and examples of the invention and, although specific terms are employed, they are used in a generic and descriptive sense only and not for the purpose of limitation, the scope of the invention being set forth in the following claims.

## Claims

1. An absorbent article (1) intended to be attached to and worn in an undergarment of a user, which absorbent article comprises:
a main body portion (2) comprising a body-facing surface (4a), an undergarment-facing surface (5a) and an absorbent core (3) positioned between the body-facing surface (4a) and the undergarment-facing surface (5a);
a plurality of fastening means (6) positioned on the undergarment-facing surface (5a) of the main body portion (2), for fastening the absorbent article (1) to the undergarment of the user;
wherein the main body portion (2) is folded along at least one folding line (X-X, Y-Y), prior to use of the absorbent article (1),
**characterized in that** each fastening means (6) has a corresponding protection means (7) for covering the fastening means, each protection means (7) being arranged with a projecting portion (7a) for facilitating removal of the protection means from the fastening means, that each of the fastening means (6) and the protection means (7) has a first portion (6a) that is distal to the at least one folding line and a second portion (6b, 7b) that is proximal to the at least one folding line, and **in that** the portion (7b) of each protection means (7) proximal to the at least one folding line (X-X, Y-Y), is coterminous with the corresponding portion (6b) of the corresponding fastening means (6) in a direction towards the at least one folding line.

2. An absorbent article according to claim 1, having two fastening means (6) and two protection means (7).

3. An absorbent article according to claim 1 or 2, having an oblong main body portion (2) with a central region (8), located between a first end region (9) and a second end region (10), the three regions being defined by two folding lines (X-X, Y-Y), wherein the fastening means (6) and the protection means (7) are located in the end regions (9, 10).

4. An absorbent article according to claim 3, wherein each projecting portion (7a) is located in an end of the protection means (7) distal to the folding lines (X-X, Y-Y) and distal to the central region (8) of the main body portion (2).

5. An absorbent article according to any of claims 1-4, wherein the portion (7b) of each protection means (7), proximal to the folding line (X-X, Y-Y) and the corresponding portion (6b) of the corresponding fastening means (6) each has a substantially rectangular shape.

6. An absorbent article according to any of claims 1-4, wherein the portion (7b) of each protection means (7), proximal to the folding line (X-X, Y-Y) and the corresponding portion (6b) of the corresponding fastening means (6) each has a rounded shape.

7. An absorbent article according to any of claims 1-5, wherein the portion (7b) of each protection means (7), proximal to the folding line (X-X, Y-Y) and the corresponding portion (6b) of the corresponding fastening means (6) each has at least one edge that is substantially parallel to its most adjacent folding line (X-X, Y-Y).

8. An absorbent article according to any of claims 1-7, wherein the fastening means (6) is an adhesive substance.

9. An absorbent article according to any of claims 1-8, wherein the fastening means (6) is arranged into a striped pattern.

## Patentansprüche

1. Absorbierender Gegenstand (1), der in einer Unterwäsche eines Benutzers befestigt und getragen werden soll, wobei der absorbierende Gegenstand Folgendes umfasst:
einen Hauptkörperabschnitt (2), umfassend eine dem Körper zugewandte Oberfläche (4a), eine der Unterwäsche zugewandte Oberfläche (5a) und einen absorbierenden Kern, der zwischen der dem Körper zugewandten Oberfläche (4a) und der der Unterwäsche zugewandten Oberfläche (5a) angeordnet ist,
mehrere Befestigungsmittel (6), die auf der der Unterwäsche zugewandten Oberfläche (5a) des Hauptkörperabschnittes (2) angeordnet sind, um den absorbierenden Gegenstand (1) an der Unterwäsche des Benutzers zu befestigen,
wobei der Hauptkörperabschnitt (2) vor der Verwendung des absorbierenden Gegenstandes (1) entlang mindestens einer Faltlinie (X-X, Y-Y) gefaltet ist,
**dadurch gekennzeichnet, dass** jedes Befestigungsmittel (6) ein entsprechendes Schutzmittel (7) zum Abdecken des Befestigungsmittels aufweist, wobei jedes Schutzmittel (7) mit einem hervorstehenden Abschnitt (7a) angeordnet ist, um das Entfernen der Schutzmittel von den Befestigungsmitteln zu erleichtern, dass jedes der Befestigungsmittel (6) und jedes der Schutzmittel (7) einen ersten Abschnitt (6a, 7a) aufweist, der fern der mindestens einen Faltlinie liegt, und einen zweiten Abschnitt (6b, 7b), der nahe der mindestens einen Faltlinie liegt, und **dadurch**, dass der Abschnitt (7b) jedes Schutzmittels (7), der nahe der mindestens einen Faltlinie (X-X, Y-Y) liegt, in einer Richtung hin zur mindestens einen Faltlinie mit dem entsprechenden Abschnitt (6b) des entsprechenden Befestigungsmittels (6) abschließt.

2. Absorbierender Gegenstand nach Anspruch 1, der zwei Befestigungsmittel (6) und zwei Schutzmittel (7) aufweist.

3. Absorbierender Gegenstand nach Anspruch 1 oder 2, der einen länglichen Hauptkörperabschnitt (2) mit einem Mittelbereich (8) aufweist, der zwischen einem ersten Endbereich (9) und einem zweiten Endbereich (10) angeordnet ist, wobei die drei Bereiche durch zwei Faltlinien (X-X, Y-Y) definiert sind, wobei die Befestigungsmittel (6) und die Schutzmittel (7) in den Endbereichen (9, 10) angeordnet sind.

4. Absorbierender Gegenstand nach Anspruch 3, wobei jeder hervorstehende Abschnitt (7a) an einem Ende der Schutzmittel (7) angeordnet ist, das fern der Faltlinien (X-X, Y-Y) und fern des Mittelbereiches (8) des Hauptkörperabschnittes (2) liegt.

5. Absorbierender Gegenstand nach einem der Ansprüche 1 bis 4, wobei der Abschnitt (7b) jedes Schutzmittels (7), der nahe der Faltlinie (X-X, Y-Y) liegt, und der entsprechende Abschnitt (6b) des entsprechenden Befestigungsmittels (6) im Wesentlichen jeweils eine rechteckige Form aufweisen.

6. Absorbierender Gegenstand nach einem der Ansprüche 1 bis 4, wobei der Abschnitt (7b) jedes Schutzmittels (7), der nahe der Faltlinie (X-X, Y-Y) liegt, und der entsprechende Abschnitt (6b) des entsprechenden Befestigungsmittels (6) im Wesentlichen jeweils eine gerundete Form aufweisen.

7. Absorbierender Gegenstand nach einem der Ansprüche 1 bis 5, wobei der Abschnitt (7b) jedes Schutzmittels (7), der fern der Faltlinie (X-X, Y-Y) liegt, und der entsprechende Abschnitt (6b) des entsprechenden Befestigungsmittels (6) jeweils mindestens einen Rand aufweisen, der im Wesentlichen parallel zu seine nächstgelegenen Faltlinie (X-X, Y-Y) verläuft.

8. Absorbierender Gegenstand nach einem der Ansprüche 1 bis 7, wobei die Befestigungsmittel (6) eine haftende Substanz sind.

9. Absorbierender Gegenstand nach einem der Ansprüche 1 bis 8, wobei die Befestigungsmittel (6) in einem Streifenmuster angeordnet sind.

## Revendications

1. Article absorbant (1) destiné à être attaché et porté dans un sous-vêtement d'un utilisateur, lequel article absorbant comprend :
une partie corporelle principale (2) comprenant une surface tournée vers le corps (4a), une surface tournée vers le sous-vêtement (5a) et un noyau absorbant (3) positionné entre la surface tournée vers le corps (4a) et la surface tournée vers le sous-vêtement (5a) ;
une pluralité de moyens de fixation (6) positionnés sur la surface tournée vers le sous-vêtement (5a) de l'utilisateur pour fixer l'article absorbant (1) au sous-vêtement de l'utilisateur ;
la partie corporelle principale (2) étant pliée le long d'au moins une ligne de pliage (X-X, Y-Y) avant l'usage de l'article absorbant (1),
**caractérisé en ce que** chaque moyen de fixation (6) comporte un moyen de protection correspondant (7) pour recouvrir le moyen de fixation, chaque moyen de protection (7) étant agencé avec une partie saillante (7a) pour faciliter le retrait du moyen de protection du moyen de fixation (6) et que le moyen de protection (7) comporte une première partie (6a) qui est distale par rapport à l'au moins une ligne de pliage et une seconde partie (6b, 7b) qui est proximale par rapport à l'au moins une ligne de pliage et que la partie (7b) de chaque moyen de protection (7) proximale par rapport à l'au moins une ligne de pliage (X-X, Y-Y) a la même terminaison que la partie correspondante (6b) du moyen de fixation correspondant (6) dans un sens allant vers l'au moins une ligne de pliage.

2. Article absorbant selon la revendication 1, comportant deux moyens de fixation (6) et deux moyens de protection (7).

3. Article absorbant selon la revendication 1 ou 2, comportant une partie corporelle principale oblongue (2) dotée d'une zone centrale (8), située entre une première zone terminale (9) et une seconde zone terminale (10), ces trois zones étant définies par deux lignes de pliage (X-X, Y-Y), le moyen de fixation (6) et le moyen de protection (7) étant situés dans les zones terminales (9, 10).

4. Article absorbant selon la revendication 3, dans lequel chaque partie saillante (7a) est située dans une extrémité du moyen de protection (7) distale par rapport à la zone centrale (8) de la partie corporelle principale (2)

5. Article absorbant selon l'une quelconque des revendications 1 à 4, dans lequel la partie (7b) de chaque moyen de protection (7) proximale par rapport à la ligne de pliage (X-X, Y-Y) et la partie correspondante (6b) du moyen de fixation correspondant (6) ont chacune une forme sensiblement rectangulaire.

6. Article absorbant selon l'une quelconque des revendications 1 à 4, dans lequel la partie (7b) de chaque moyen de protection (7) proximale par rapport à la ligne de pliage (X-X, Y-Y) et la partie correspondante (6b) du moyen de fixation correspondant (6) ont chacune une forme sensiblement arrondie.

7. Article absorbant selon l'une quelconque des revendications 1 à 5, dans lequel la partie (7b) de chaque moyen de protection (7) proximale par rapport à la ligne de pliage (X-X, Y-Y) et la partie correspondante (6b) du moyen de fixation correspondant (6) ont chacune au moins un bord qui est sensiblement parallèle à sa ligne de pliage la plus adjacente (X-X, Y-Y).

8. Article absorbant selon l'une quelconque des revendications 1 à 7, dans lequel le moyen de fixation (6) est une substance adhésive.

9. Article absorbant selon l'une quelconque des revendications 1 à 8, dans lequel le moyen de fixation (6) est disposé en forme de bande.
